(19) Europäisches Patentamt
European Patent Office
Office européen des brevets

(11) Veröffentlichungsnummer: **0 008 656**
**A2**

# (12) EUROPÄISCHE PATENTANMELDUNG

(21) Anmeldenummer: 79102599.2

(22) Anmeldetag: 23.07.79

(51) Int. Cl.³: C 12 P 1/00
C 12 P 21/06, C 12 P 19/14
C 12 N 11/08, A 23 C 9/12

(30) Priorität: 13.09.78 DE 2839737

(43) Veröffentlichungstag der Anmeldung:
19.03.80 Patentblatt 80/6

(84) Benannte Vertragsstaaten:
BE CH FR GB IT NL SE

(71) Anmelder: Röhm GmbH
Kirschenallee
D-6100 Darmstadt(DE)

(72) Erfinder: Reimerdes, Ernst H., Dr.
Sandkoppel 26
D-2300 Kronshagen(DE)

## (54) Verfahren zur enzymatischen Umsetzung mittels trägergebundener Enzyme.

(57) Die Erfindung betrifft ein Verfahren zur enzymatischen Umsetzung eines biologischen Substrats in wäßriger Lösung, die neben dem Substrat zu hydrophober Wechselwirkung befähigte Begleitstoffe enthält mittels eines gegenüber dem Substrat aktiven Enzyms, das bei hydrophober Wechselwirkung mit den Begleitstoffen gehemmt wird, wobei die enzymatische Umsetzung in Anwesenheit der hydrophoben Begleitstoffe durchgeführt wird und das eingesetzte Enzym adsorptiv an einem hydrophob modifizierten, porösen Träger mit hydrophiler Matrix gebunden ist.

EP 0 008 656 A2

Croydon Printing Company Ltd.

Verfahren zur enzymatischen Umsetzung mittels trägergebundener Enzyme

Die Erfindung betrifft ein Verfahren zur enzymatischen Umsetzung von biologischen Substraten in wäßriger Lösung mittels trägergebundener Enzyme. Bei derartigen Verfahren wird in manchen Fällen ein rascher Aktivitätsverlust des gebundenen Enzyms beobachtet, beispielsweise beim Abbau von Milchzucker in Milch oder Molke mittels einer kovalent an einen Träger gebundenen Lactase. Wenn dagegen eine reine Milchzuckerlösung an der gleichen trägergebundenen Lactase umgesetzt wird, so ist kein beschleunigter Aktivitätsverlust festzustellen. Diese Beobachtungen gehen auf W.H. Pitcher, (Proceedings Whey Products Conference 1974, USDA Publ. Errc. Nbr. 3996; 20th International Dairy Congress, Paris, 1978, S. 952 - 953.) zurück. Eine gleichartige Hemmung tritt auch beim Einsatz der ungebundenen Lactase in Milch oder Molke ein, nicht dagegen in einer reinen Milchzuckerlösung. Es wurde festgestellt, daß die Hemmung auf eine hydrophobe Wechselwirkung von hydrophoben Bereichen des Enzyms mit bestimmten hydrophoben Begleitstoffen, die hydrophobe Strukturelemente enthalten, zurückzuführen ist. Im Falle der Milch oder der Molke handelt es sich um Kaseine. Sie lagern sich an hydrophobe Stellen der Enzymoberfläche an unter Bindung eines gegenüber Lactose inaktiven Komplexes.

Der Abbau von Milchzucker in Milch und insbesondere in Molke stellt ein technologisch bedeutsames Problem dar. Milchzucker ist für viele Menschen, namentlich in Entwicklungsländern, unverträglich; darüber hinaus ist er schwer löslich und von geringer Süßkraft. Um den Milchzucker in Milch verträglich zu machen oder um den in Molke enthaltenen Milchzucker verwerten zu können, wird eine enzymatische Spaltung in Glucose und Galactose angestrebt. Diese Zucker sind nicht nur voll verträglich, sondern auch von hoher Süßkraft und guter Löslichkeit. Wegen des erwähnten raschen Aktivitätsverlusts war es bisher mit einem wirtschaftlich vertretbaren Aufwand nicht möglich, den Milchzucker mittels freier oder trägergebundener Lactase unmittelbar in Milch oder Molke abzubauen. Vielmehr war es im Falle der Molke notwendig, die Eiweißbestandteile durch Ultrafiltration zu entfernen, das Filtrat zu entionisieren und den enzymatischen Abbau, zweckmäßig mit kovalent trägergebundener Lactase, in dem gereinigten Filtrat durchzuführen. Dieser erhebliche technische Aufwand ist unwirtschaftlich.

Ähnlich wie im Falle der Kaseine in Milch und Molke beobachtet man auch bei anderen enzymatischen Umsetzungen gelegentlich eine rasche Enzyminaktivierung infolge einer hydrophoben Wechselwirkung zwischen dem Enzym und hydrophoben Begleitstoffen des Substrats, deren Abtrennung in der Regel nur mit großem technischem Aufwand möglich ist.

Der Erfinder hatte sich die Aufgabe gestellt, ein Verfahren zur enzymatischen Umsetzung von biologischen Substraten in wäßriger Lösung mittels trägergebundener Enzyme in Gegenwart der Begleitstoffe durchzuführen, die schon mit dem ungebundenen Enzym in hydrophobe Wechselwirkung treten. Überraschenderweise tritt die rasche Hemmung nicht ein, wenn die enzymatische Umsetzung mit einem Enzym durchgeführt wird, das adsorptiv an einen hydrophob modifizierten, porösen Träger mit hydrophiler Matrix gebunden ist. Dieses Ergebnis ist überraschend, weil die hydrophobe adsorptive Bindung zwangsläufig gerade an denjenigen Stellen der Enzymoberfläche stattfinden muß, an denen die adsorptive Bindung mit Kasein oder ähnlich wirkenden Begleitstoffen zur Aktivitätshemmung führt. Überraschenderweise verhindert die adsorptive Bindung an den hydrophoben Träger die Wechselwirkung der hydrophoben Enzymoberfläche mit z.B. Kasein, ohne die Aktivität herabzusetzen, und schafft dadurch die Voraussetzung, unter der das Enzym überhaupt über lange Zeit in Gegenwart der hydrophob wechselwirkenden Begleitstoffe aktiv bleiben kann.

Wenn auch die Spaltung von Lactose in kaseinhaltiger Molke oder Milch mit Hilfe einer adsorptiv an einen hydrophobierten Träger gebundenen Lactase die bevorzugte Ausführungsform der Erfindung darstellt, so kann das zugrundeliegende Prinzip auf eine Vielzahl anderer Umsetzungen angewendet werden. Alle Enzyme besitzen

hydrophobe Stellen an ihrer Oberfläche und können mit anderen hydrophoben Begleitstoffen - in der Regel unter Verlust der enzymatischen Aktivität - in Wechselwirkung treten. Beispielsweise können im Sinne der Erfindung enzymatische Umsetzungen mit Proteasen, Lipasen, Invertase oder Isomerase durchgeführt werden. Zur Umsetzung mit den adsorptiv an einen Träger gebundenen Enzymen eignen sich die wäßrigen Lösungen der entsprechenden Substrate, d.h. Lösungen von Sacchariden, Disacchariden, Polysacchariden, Proteinen, Lipiden usw., wobei es nicht darauf ankommt, ob die genannten Substrate im strengen Sinne gelöst sind oder in mehr oder weniger kolloidaler Verteilung vorliegen. Kohlenhydrate und Proteine sind die bevorzugten Substrate.

Die hydrophob wechselwirkenden Begleitstoffe sind im Falle von Milch und Molke vor allem α-, β-, γ-, und κ-Kaseine, von denen sich die β- und γ-Kaseine durch eine besonders starke Inaktivierungswirkung auszeichnen. Auch manche Eiweißhydrolysate haben eine starke Inaktivierungswirkung.

Die Inaktivierung durch hydrophobe Wechselwirkung, deren Ausschaltung das Ziel des vorliegenden Verfahrens ist, gibt sich bei der Anwendung eines kovalent an einen Träger gebundenen Enzyms dadurch zu erkennen, daß die Halbwertzeit der Enzymaktivität bei der Umsetzung mit der hydrophobe Begleitstoffe enthaltenden Substratlösung höchstens noch die Hälfte, häufiger nur noch ein Viertel oder

weniger der Halbwertzeit beträgt, die an einer reinen Lösung des Substrats gefunden wird. So beträgt (nach Pitcher) die Halbwertzeit von kovalent immobilisierter ß-Galactosidase aus Aspergillus niger gegenüber einer 5 %igen Lactoselösung etwa 125 Tage, bei Einwirkung von Molke unter sonst gleichen Bedingungen nur etwa 4 Tage.

Die hydrophobe adsorptive Bindung von Proteinen, besonders Enzymen, an hydrophobierte Träger ist an sich bekannt und nicht Gegenstand der vorliegenden Erfindung. K.D. Caldwell, R. Axén, M. Bergwall, J. Porath, (Biotechnology and Bioengineering, Bd. XVIII, Seiten 1573 bis 1604) beschreiben das Prinzip dieser Bindung am Beispiel von ß-Amylase und Amyloglucosidase aus Aspergillus niger, die an hydrophobierte Agarose oder Sepharose gebunden werden. Nach L.G. Butler (Chemistry and Engineering News, 30.5.1977, Seite 23) erhält man eine besonders feste Bindung von Proteinen, insbesondere Enzymen, an Phenoxyacetylcellulose.

Als Trägermatrix werden erfindungsgemäß solche Stoffe verwendet, die auch bei anderen biochemischen Verfahren günstige technische Einsatzbedingungen aufweisen. Die Stoffe sollen hydrophil sein, um der wäßrigen Substratlösung ein leichtes Eindringen zu gestatten. Vorteilhaft ist eine poröse Struktur, wobei die Poren eine zur Aufnahme des Substrats ausreichende Größe haben müssen.

Dadurch steht eine hohe innere Oberfläche für die Bindung des Enzyms zur Verfügung. Die Porosität ist als solche nur schwer meßbar und offenbart sich in der Regel nur indirekt durch eine hohe Beladungsdichte. Für technische Durchflußreaktoren sind eine hohe mechanische Stabilität der Trägerpartikel und gute Durchflußeigenschaften wichtig. Annähernd kugelförmige Teilchen mit Durchmessern von 0,1 bis 3 mm sind besonders vorteilhaft.

Selbstverständlich muß die Trägermatrix gegenüber dem bei der Umsetzung verwendeten Enzym sowie gegenüber allen übrigen Bestandteilen der umzusetzenden Substratlösung hinreichend beständig sein.

Es gibt eine Reihe gebräuchlicher Trägermatrizes, die diese Forderungen weitgehend oder vollständig erfüllen. Dazu gehören vernetzte Agarose und Dextrangele, Cellulose, Hydroxyäthylcellulose, Mischpolymerisate von Acrylamid mit vernetzenden Comonomeren, wie z.B. Methylenbisacrylamid. Weiterhin gibt es anorganische Trägermaterialien, insbesondere Glaskügelchen, zum Teil mit Porenstruktur. Die erwünschte Porenstruktur und mechanische Stabilität läßt sich, beispielsweise gemäß dem Verfahren der DE-AS 2 343 633 durch Mischpolymerisation hydrophiler Monomerer, wie Acrylamid oder Hydroxyäthylmethacrylat, in Gegenwart eines mehrfunktionellen, vernetzenden Comonomeren in Form eines Perlpolymerisats erzeugen. Dabei wirkt sich die Anwesenheit eines Quellungsmittels und eines hohen Anteils des Vernetzungsmittels vorteilhaft auf die Porenstruktur und mechanische Beständigkeit aus.

Hydrophile, poröse Trägermatrizes dieser Art werden bereits zur Herstellung von kovalent immobilisierten Enzymen eingesetzt. Sie werden dazu entweder nachträglich aktiviert, so daß sie gegenüber Proteinen bindungsaktiv sind - geeignete Aktivierungsmittel sind z.B. Bromcyan, Bisepoxide oder Dialdehyde - oder sie werden von vorneherein unter Mitverwendung von gegenüber Proteinen reaktiven Comonomeren, wie Maleinsäureanhydrid,+) Glycidylmethacrylat und dergleichen, hergestellt. Um Träger für die hydrophobe adsorptive Proteinbindung zu erzeugen, werden die bindungsaktiven Matrizes nicht unmittelbar mit dem Enzym, sondern mit einer hydrophobierenden Substanz umgesetzt, die dabei vorzugsweise kovalent an die Matrix gebunden ist. Geeignete hydrophobe Gruppen sind Alkylreste,bevorzugt solche mit wenigstens 4 Kohlenstoffatomen, sowie aromatische Gruppen. Vorzugsweise enthalten die Alkylreste, die geradkettig oder verzweigt sein können, nicht mehr als 18 C-Atome. Der Bereich von 6 bis 12 C-Atomen stellt ein Optimum dar. Unter den aromatischen Gruppen sind Phenyl-, Toluyl-, andere Alkylphenyl- und Benzylgruppen bevorzugt, jedoch kommen auch Naphthylgruppen und andere mehrkernige aromatische Reste in Betracht. Die zur Hydrophobierung des Trägers eingesetzten Verbindungen müssen neben der

+) Methacrylsäureanhydrid, Acrylsäureanhydrid,

hydrophoben Gruppe eine gegenüber dem aktivierten Träger bindungsaktive Gruppe aufweisen. Mit Bromcyan aktivierte Träger und solche mit Carbonsäureanhydrid- oder Carbonsäurechloridgruppen reagieren z.B. mit Amino- oder Hydroxygruppen. Träger mit Epoxidgruppen reagieren außer mit den bereits genannten Gruppen auch mit Carboxylgruppen. Zur Hydrophobierung eignen sich demzufolge - je nach der bindungsaktiven Gruppe des Trägers - höhere aliphatische oder aromatische Alkohole, primäre Amine, Merkaptane oder Carbonsäuren.

Die günstigste hydrophobe Bindungsfähigkeit wird in vielen Fällen noch nicht erreicht, wenn die hydrophobe Gruppe unmittelbar an die Hauptkette der Trägermatrix gebunden ist. Eine gewisse Beweglichkeit der hydrophoben Gruppe fördert ihre Bindungsfähigkeit.Man verwendet daher mit besonderem Vorteil solche hydrophobierenden Verbindungen, bei denen nach der Umsetzung mit dem reaktiven Trägermaterial eine Kette von wenigstens 3, vorzugsweise 4 bis 10 Atomen zwischen der Hauptkette des Trägermaterials und der hydrophoben Gruppe angeordnet ist. Eine solche, häufig als "Spacer" bezeichnete Zwischengruppe entsteht z.B., wenn ein Vinylcopolymerisat mit Einheiten des Glycidylacrylats oder -methacrylats als Matrix mit Benzylalkohol, Hexanol oder Octylmerkaptan hydrophobiert wird. Der Einfluß von "Spacern" auf das Affinitätsverhalten von hydrophilen Trägermaterialien mit

bindungsaktiven Gruppen gegenüber Proteinen hat P. O'Carra (Industrial Aspects of Biochemistry, herausgegeben von B. Spencer, 1974, Seite 107 bis 134) für das Gebiet der Affinitätschromatographie untersucht. Seine Erkenntnisse lassen sich sinngemäß auf die erfindungsgemäß verwendeten hydrophobierten Trägermaterialien übertragen.

In manchen Fällen lassen sich hydrophobierte Träger ohne besondere Aktivierungsstufe durch unmittelbare Umsetzung der hydrophilen Matrix mit einer hydrophobierenden Verbindung erzeugen. Das gilt z.B. für die Umsetzung von Cellulose mit Phenoxyacetylchlorid. Bei der Herstellung vollsynthetischer Trägermatrizes, z.B. aus Acrylamid oder Hydroxymethylmethacrylat und vernetzenden Comonomeren, besteht grundsätzlich auch die Möglichkeit, Comonomere mit hydrophilen Seitengruppen bei der Polymerisation mitzuverwenden, wie z.B. höhere Alkylester der Acryl- oder Methacrylsäure oder Phenoxyalkylester dieser Säure. Es sei jedoch wiederholt, daß die Herstellung hydrophob modifizierter Trägermaterialien zum Stand der Technik gehört und dem Fachmann zahlreiche analoge Arbeitsweisen zu den hier beispielhaft beschriebenen Hydrophobierungsmethoden zur Verfügung stehen.

Die optimale Menge an hydrophoben Gruppen läßt sich am einfachsten durch eine Versuchsreihe

ermitteln. Da der Träger an seiner zugänglichen Oberfläche hydrophob modifiziert wird, läßt sich die optimale Beladungsdichte nicht auf das Gewicht des Trägermaterials beziehen. Da andererseits auch die zugängliche Oberfläche in der Regel nicht meßbar ist, läßt sich auch für die optimale Beladungsdichte kein Zahlenwert ermitteln. Für die Praxis liegt eine untere Grenze der Hydrophobierung da, wo die adsorptive Bindungsfähigkeit gegenüber dem Enzym unter eine technisch sinnvolle Grenze sinkt. Die Obergrenze der Hydrophobierung ist erreicht, wenn die Hydrophilie der Matrix so stark herabgesetzt wird, daß die wäßrige Substratlösung nicht mehr ausreichend in die Poren einzudringen vermag. Die Hydrophobierung braucht nur so stark zu sein, um gerade die technisch erwünschte Enzymmenge binden zu können. Diese Enzymmenge kann z.B. im Bereich von 0,05 bis 2 %, vorzugsweise 0,1 bis 1 %, bezogen auf das Gewicht der Polymermatrix, liegen. Dies entspricht einer Enzymmenge, die auch bei der kovalenten Bindung von Enzymen an aktivierte Träger erreicht wird. Es ist nicht vorteilhaft, die Enzymbeladung bis zur vollständigen Besetzung der verfügbaren Oberfläche zu steigern, weil es dann zur sterischen Behinderung der Enzymmoleküle untereinander kommt. Die Zahl der bindungsaktiven hydrophoben Gruppen an dem Trägermaterial liegt daher im allgemeinen in der gleichen Größenordnung wie die Zahl der aktivierten Gruppen an kovalent bindenden Trägern. Man kann daher von derartigen Trägern ausgehen und die reaktiven Gruppen mit

einer damit reaktionsfähigen hydrophoben Verbindung umsetzen und gelangt auf diese Weise zu einer für praktische Zwecke ausreichenden Hydrophobierung.

An einem vernetzten Acrylamidcopolymerisat, an dessen Aufbau 40 Gew.-% Glycidylmethacrylat beteiligt sind, lassen sich z.B. 1 bis 2 % reaktionsfähiger Oxiran-Sauerstoff nachweisen, was etwa einem Viertel bis der Hälfte der eingesetzten Oxiranmenge entspricht. Die Umsetzung eines solchen Trägerharzes mit etwa 2 bis 6 Gew.-% Benzylalkohol ergibt ein hydrophobiertes Harz, an das sich etwa 200 IU/g einer Lactase aus einer Kultur von Escherichia coli (berechnet als aktives Enzym) binden läßt. Wie viele hydrophobe Gruppen zur Bindung eines Enzymmoleküls erforderlich sind, ist nicht bekannt, jedoch dürfte es bei der Verwendung niederer Alkylgruppen, wie Methyl-, Äthyl-, Propyl-, oder Isopropylgruppen als hydrophober Komponente einer Mehrzahl derartiger Gruppen bedürfen, um ein Enzymmolekül zu binden.

Wenn auch das Prinzip der hydrophoben Bindung von Proteinen allgemein gültig ist, so ist nicht jeder beliebige Träger zur Bindung jedes beliebigen Enzyms in der für ein technisches Verfahren erwünschten Aktivität geeignet. Um den optimalen Träger für ein bestimmtes Enzym zu ermitteln, ist es zweckmäßig, sowohl die hydrophile Matrix als auch die hydrophobierende Verbindung zu variieren. Bei günstiger Abstimmung zwischen Binderharz und

Enzym läßt sich eine nahezu quantitative Bindung des Enzyms ohne nennenswerten Aktivitätsverlust erreichen. Die Bindung ist nahezu irreversibel, so daß Enzymverluste durch Desorption während der Anwendung kaum zu beobachten sind. Wenn nach längerer Betriebsdauer die Aktivität des gebundenen Enzyms spürbar abgesunken ist, läßt es sich mit einer Harnstofflösung desorbieren, oder es kann nach einer Denaturierung mit Säuren oder Laugen durch proteolytische Enzyme abgebaut werden. Der gereinigte Träger kann mehrfach neu beladen werden.

Das Enzym wird innerhalb seines Stabilitätsbereiches, vorzugsweise bei einem pH-Wert innerhalb seines Aktivitätsbereiches aus einer wäßrigen Lösung auf den Träger aufgebracht. Sowohl zur Beladung als auch zur späteren Anwendung des beladenen Trägers kann man sich sowohl des kontinuierlichen Säulendurchlaufverfahrens als auch eines ansatzweisen Verfahrens im Rührkessel bedienen. Im ersteren Fall läßt man die gepufferte Enzymlösung über eine mit dem Harz gefüllte Säule laufen, wobei das Enzym adsorbiert wird. Im letzteren Fall wird das Trägerharz mit der Pufferlösung bis zur erfolgten Adsorption gerührt und anschließend abfiltriert. Die Anwendung der Erfindung wird nachfolgend am Beispiel der Lactosespaltung in Molke näher erläutert, ohne daß die dort beschriebene Arbeitsweise auf die Lactosespaltung beschränkt sein soll.

Eine weitere Anwendung der Erfindung ist die Behandlung von Milch mit trägergebundenen Labenzymen. Aus tierischen Organen gewonnene Labenzyme, wie Chymosin oder Rennin, lassen sich in hoher Aktivitätsausbeute an hydrophobierte Träger binden und erfindungsgemäß zur Dicklegung von Milch einsetzen. Auch nicht-tierische Labaustauschstoffe, beispielsweise aus Mikroorganismen, kommen in trägergebundener Form für dieses Verfahren in Betracht.

## Beispiele

### A) Hydrophobierung des Trägerharzes

Als Trägerharz wird ein perlförmiges Mischpolymerisat eingesetzt, das nach dem Verfahren der DE-AS 2 343 633, jedoch aus einem Monomerengemisch der Zusammensetzung

| | |
|---|---|
| 12 | Gew.-Teile N,N'-Methylenbis-methacrylamid |
| 5,7 | Gew.-Teile Methacrylamid |
| 7 | Gew.-Teile Glycidylmethacrylat |
| 6 | Gew.-Teile Allylglycidyläther |

hergestellt worden ist und 1,5 Gew.-% reaktiven Oxiran-Sauerstoff enthält. 20 g des Harzes werden mit 200 ml einer 10 %igen Lösung von Benzylalkohol in Dimethylformamid 96 Stunden bei 60° unter Feuchtigkeitsausschluß umgesetzt. Anschließend wird die Flüssigkeit abgesaugt und das Harz dreimal mit je 750 ml Dimethylformamid und einmal mit 750 ml Aceton gewaschen. Das so gereinigte Harz wird 24 St. bei Raumtemperatur mit 0,2 m $NaH_2PO_4$-Pufferlösung behandelt, anschließend mit 1 m NaCl-Lösung gewaschen und mit 0,1 m $K_2HPO_4$-Pufferlösung von pH 6,2 äquilibriert.

B) Bindung des Enzyms

Je Gramm des feuchten, hydrophobierten Trägerharzes werden 5 bis 10 mg eines Lactase-Präparates aus einer Kultur von Escherichia coli mit einer Aktivität von 5000 IU/g eingesetzt. Das Enzym ist in einer Konzentration von 10 mg / 25 ml in 0,1 m $K_2HPO_4$-Pufferlösung von pH 6,2 gelöst und gegen die gleiche Pufferlösung dialysiert. Nach 10 bis 20 min ist das Enzym quantitativ ohne Aktivitätsverlust an den Träger gebunden. Man findet eine Aktivität von 25 bis 50 IU/g (bezogen auf Feuchtgewicht). Die Beladung läßt sich bis auf 220 IU/g steigern, wenn man entsprechend mehr Enzym einsetzt.

C) Lactose Spaltung

1) Zur Spaltung einer 4,5 %igen Lactoselösung in 0,1 m Phosphatpuffer von pH 7,5 wird ein Säulenreaktor von 2 cm Innendurchmesser mit einer Schütthöhe von 5,5 cm des gemäß B) erhaltenen perlförmigen Lactase-Harzes eingesetzt. Man läßt bei Raumtemperatur 50 ml/h der Lactoselösung durchlaufen und erhält eine Spaltung von 85 %.

2) Unter den gleichen Bedingungen läßt man 50 ml/h Molke mit pH 7,5 durch den Reaktor laufen und erhält eine Spaltung von 65 % der darin enthaltenen Lactose. Innerhalb von 14 Tagen ist kein Aktivitätsabfall festzustellen. Der Umsetzungsgrad nimmt zu, wenn die Durchflußgeschwindigkeit vermindert wird.

3) Über den gleichen Reaktor läßt man 50 ml/h Magermilch von pH 7,5 laufen und erhält eine Umsetzung der darin enthaltenen Lactose von 75 %. Innerhalb von 10 Tagen tritt kein Aktivitätsabfall ein.
Man erhält vergleichbare Ergebnisse, wenn man Lactoselösung, Milch oder Molke 1 bis 2 Std. bei Raumtemperatur mit dem Enzymharz rührt und die Substratlösung anschließend abfiltriert.

Verfahren zur enzymatischen Umsetzung mittels trägergebundener Enzyme

Patentansprüche

1. Verfahren zur enzymatischen Umsetzung eines biologischen Substrats in wäßriger Lösung, die neben dem Substrat zu hydrophober Wechselwirkung befähigte Begleitstoffe enthält, mittels eines gegenüber dem Substrat aktiven Enzyms, das bei hydrophober Wechselwirkung mit den Begleitstoffen gehemmt wird,

dadurch gekennzeichnet,

daß die enzymatische Umsetzung in Gegenwart der hydrophoben Begleitstoffe durchgeführt wird und das eingesetzte Enzym adsorptiv an einen hydrophob modifizierten, porösen Träger mit hydrophiler Matrix gebunden ist.

2. Verfahren nach Anspruch 1, dadurch gekennzeichnet, daß der hydrophob wechselwirkende Begleitstoff ein Kasein ist.

3. Verfahren nach den Ansprüchen 1 und 2, dadurch gekennzeichnet, daß die wäßrige Lösung von biologischen Substraten Molke oder Milch ist.

4. Verfahren nach den Ansprüchen 1 bis 3, dadurch gekennzeichnet, daß die biologischen Substrate Kohlenhydrate und/oder Proteine sind.

5. Verfahren nach Anspruch 4, dadurch gekennzeichnet, daß das biologische Substrat eine Lactose ist.

6. Verfahren nach den Ansprüchen 1 bis 5, dadurch gekennzeichnet, daß eine trägergebundene Lactase, Proteinase, Lipase, Invertase oder Isomerase eingesetzt wird.

7. Verfahren nach Anspruch 6, dadurch gekennzeichnet, daß eine trägergebundene Lactase eingesetzt wird.

8. Verfahren nach Anspruch 7, dadurch gekennzeichnet, daß eine trägergebundene Lactase aus einer Kultur von Escherichia coli eingesetzt wird.

9. Verfahren nach Anspruch 7, dadurch gekennzeichnet, daß eine trägergebundene Lactase aus einer Kultur von Bac. subtilis eingesetzt wird.

10. Verfahren nach Anspruch 7, dadurch gekennzeichnet, daß eine trägergebundene Lactase aus einer Kultur von Aspergillus niger eingesetzt wird.

11. Verfahren nach Anspruch 7, dadurch gekennzeichnet, daß eine trägergebundene Lactase aus einer Kultur von Saccharomyces lactis eingesetzt wird.

12. Verfahren nach den Ansprüchen 1 bis 5, dadurch gekennzeichnet, daß ein trägergebundenes Labenzym eingesetzt wird.

13. Verfahren nach Anspruch 11, dadurch gekennzeichnet, daß das Labenzym aus tierischen Organen stammt.

14. Verfahren nach den Ansprüchen 1 bis 13, dadurch gekennzeichnet, daß das Enzym adsorptiv an einen porösen, hydrophilen Träger, der durch kovalente Bindung von Verbindungen mit geradkettigen oder verzweigten, vorzugsweise wenigstens 4 Kohlenstoffatome enthaltenden Alkylgruppen oder mit aromatischen Gruppen an die hydrophile Matrix hydrophobiert ist, gebunden ist.

15. Verfahren nach Anspruch 14, dadurch gekennzeichnet, daß der hydrophobierende Träger ein vernetztes Mischpolymerisat von Acrylamid mit eine hydrophobierende Gruppe tragenden Comonomeren ist.

16. Verfahren nach Anspruch 15, dadurch gekennzeichnet, daß das Mischpolymerisat als hydrophobierendes Comonomer Einheiten der Struktur

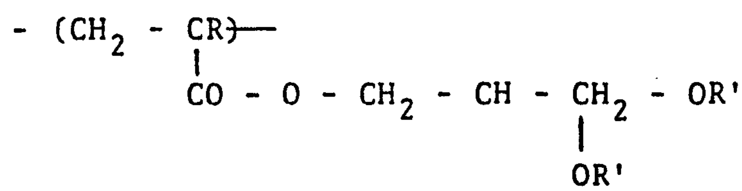

worin R ein Wasserstoffatom oder eine Alkylgruppe und der eine Rest R' ein Wasserstoffatom und der andere Rest R' eine Alkyl-, Aryl- oder Aralkylgruppe ist, in der Hauptkette der hydrophilen Matrix enthält.